# EUROPÄISCHE PATENTSCHRIFT

(11) **EP 0 841 954 B1**
(45) Veröffentlichungstag und Bekanntmachung des Hinweises auf die Patenterteilung: **01.09.1999**
(21) Anmeldenummer: 95936586.7
(22) Anmeldetag: 03.11.1995
(51) Int. Cl.: A61L 2/18, A61L 2/04, A61K 35/16

(54) **VERFAHREN ZUR HERSTELLUNG EINES VIRUSSICHEREN, THERAPEUTISCHEN, BIOLOGISCHEN PRÄPARATES**
METHOD FOR THE PRODUCTION OF VIRUSSAFE, THERAPEUTIC, BIOLOGICAL PREPARATIONS
PROCEDE DE PREPARATION DE PRODUITS BIOLOGIQUES ET THERAPEUTIQUES SAINS ET SAUFS DE CONTAMINATION PAR DES VIRUS

(30) Priorität: 01.08.1995 DE 19528221
(43) Veröffentlichungstag der Anmeldung: 20.05.1998
(73) Patentinhaber: Blutspendedienst der DRK-Landesverbände Nordrhein und Westfalen-Lippe GmbH, 58097 Hagen (DE)
(72) Erfinder: FAUCOMPRE, Annick, F-59700 Marcq-en-Baroeul (FR); WÖBER, Günter, D-58099 Hagen (DE)
(74) Vertreter: Hoffmeister, Helmut, Dr. Dipl.-Phys.
(86) Internationale Anmeldenummer: EP9504309
(87) Internationale Veröffentlichungsnummer: WO9704815

(56) Entgegenhaltungen:
- EP-A- 0 099 445
- WO-A-94/13329
- DE-A- 4 318 435

## Beschreibung

Die Erfindung betrifft ein Verfahren zur Herstellung eines virussicheren, therapeutischen, biologischen Präparates aus menschlichem Blutplasma unter Anwendung eines Detergens, unter Erhaltung von mindestens 50% der biologischen Aktivität und unter Anwendung einer Detergensbehandlung, an deren Schluß die Detergentien entfernt werden.

Unter biologischen Präparaten aus Humanblut werden Produkte aus menschlichem Blut oder Plasma verstanden, die zur therapeutischen, prophylaktischen oder diagnostischen Anwendung bestimmt sind. Solche Produkte können Enzyme, Proenzyme, einschließlich Gerinnungsfaktoren, Enzyminhibitoren, Immunglobuline, Albumin, Plasminogen, Fibrinogen, Fibronectin oder Plasma umfassen. Ausgehend von infizierten Spendern können derartige Produkte Viren enthalten, die vor der Verabreichung an dritte Personen entfernt oder inaktiviert werden müssen.

Es ist eine umfangreiche patentrechtliche und wissenschaftliche Literatur vorhanden, in der Inaktivierungsverfahren beschrieben werden, so daß nur einige prägnante Beispiele herangezogen werden sollen. EP-A-0 050 061 (Erfinder: Shanbrom) beschreibt ein Verfahren zur Verringerung oder Beseitigung von Virus-Infektiosität in biologischen Produkten, das die Stufe der Zugabe eines Amphiphils umfaßt. Die Produkte werden durch einen verlängerten Kontakt von mindestens 30 Minuten mit einer Lösung oder Suspension von 0,25 bis 10 Gew.% eines nicht-denaturierenden Amphiphils behandelt. Anschließend wird das Amphiphil mit den die Pyrogenizität bewirkenden Substanzen entfernt. Diese Behandlung zielt auf auf die Inaktivierung von Hepatitis B-Viren und non-A, non-B Hepatitis-Viren ab, die membranumhüllt sind.

Ein gegenüber EP-A-0 050 061 verbessertes Verfahren ist in der EP-A-0 131 740 (Erfinder: Neurath, Horowitz) beschrieben. Das Verfahren dient dazu, eine ein labiles Protein enthaltende Zusammensetzung im wesentlichen frei von lipidumhüllten Viren zu machen, ohne daß eine wesentliche Proteindenaturierung herbeigeführt wird. Das Verfahren besteht darin, daß die Zusammensetzung in Kontakt gebracht wird mit einer wirksamen Mengen eines Di- oder Trialkylphosphats zusammen mit einem Tensid als Lösungsvermittler für eine Zeitdauer, die ausreichend ist, um die Zusammensetzung frei von lipidenthaltenden Viren zu machen, ohne wesentliche Proteindenaturierung herbeizuführen. Üblicherweise wird bei einer Temperatur von 0 bis 70°C gearbeitet. Es ist erforderlich, die labilen Proteine bei Erhitzen der Lösung zu stabilisieren.

Die Behandlung von Detergentien hat allgemein den Nachteil, daß sie lediglich auf membranumhüllte Viren abzielt. Wenn ein Detergens in einer Konzentration über der micellbildenden Konzentration (CMC) vorliegt, werden lipidhaltige Membranen solubilisiert und das Virus inaktiviert. Demgegenüber können Viren, die an sich keine lipidhaltige Membran aufweisen, wie zum Beispiel Hepatitis A-Virus, durch eine Detergensbehandlung aus Vesikeln freigesetzt und damit aktiviert anstelle von inaktiviert werden.

Für die volle Wirksamkeit der vorgenannten Verfahren ist außerdem der Zusatz einer viruziden Chemikalie, z.B. Tri-n-butylphosphat (TNBP) unerläßlich (B. Horowitz et al, Transfusion 25, 516 - 522, 1985).

Ebenfalls ist aus der Literatur bekannt, daß das Vaccinia-Virus, ein Mitglied der Poxviren, überraschenderweise selbst gegen das Solvens-Detergens-Verfahren nach Neurath/Horowitz resistent ist, obwohl es ebenfalls ein membranumhülltes Virus ist (H. Hart et al, Poster bei Scotblood '92, University of Sterling, Juni 1992 "Model Virus Studies of Solvent/Detergent Treatment used in the Production of High-Potency Factor VIII"). Die Autoren vermuten, daß diese Behandlung für die Hülle des Vaccinia-Virus zu mild sei.

Siehe auch die Entgegen haltungen: WO-A-94/13329; insbesondere EP-A-0 099 445; und DE-A-4 318 435. Es stellt sich daher die Aufgabe, ein Verfahren anzugeben, das grundsätzlich die bewährte Technologie einer Detergensbehandlung aufnimmt, dem eine Wärmebehandlung folgen oder vorausgehen kann, jedoch umfassend sowohl membranumhüllte als auch nicht-membranumhüllte Viren inaktiviert und außerdem dabei Detergentien verwendet, die relativ einfach nach der Behandlung wieder entfernt werden können.

Die Aufgabe wird gelöst durch ein Verfahren, das alle Merkmale des Anspruchs 1 aufweist. Bevorzugt Merkmale sind in den abhängigen Ausprüchen definiert.

Als nicht-ionischer Detergentien im Sinne der Erfindung seien genannt:
- Triethylenglycol-mono-octylether,
- N-(D-gluco-2,3,4,5,6-pentahydroxyhexyl)-N-methyloctanamid,
- 1-Thio-n-octyl-β-D-glucopyranosid,

Derartige Produkte werden im Handel vertrieben. Einige der Handelsnamen sind: MEGA-8 N-(D-gluco-2,3,4,5,6-pentaydroxyhexyl)-N-methyloctanamid

Als bevorzugte Verfahrensparameter können angesehen werden: Konzentration des Detergens zwischen 5g/l bis 20g/l; Einwirkungsdauer des Detergens: vorzugsweise 10 bis 20 Minuten; Behandlungstemperatur vorzugsweise 25°C.

Die Behandlungsdauer ist, wie ersehbar, wesentlich geringer als 30 Minuten zu halten. Die Behandlungs- und die Einwirkungsdauer können abrupt beendet werden, indem die Konzentration des Detergens durch Zugabe von Wasser soweit verdünnt wird, daß die CMC (kritsche Micellkonzentration) unterschritten und damit die Einwirkung abgebrochen wird.

Das in der Lösung verbleibende Detergens kann durch Dialyse oder Diafiltration entfernt werden.

Der Detergensschritt kann mit einem Pasteurisierungs(Erwärmungs)schritt kombiniert werden. Eine umfangreiche Literatur ist vorhanden, die sich mit der Inaktivierung von infektiösen Agentien durch Erhitzen von Blutprodukten befaßt. Die verschiedenen Verfahren umfassen:
- Erhitzen der Blutprodukte in wässeriger Lösung in Anwesenheit von Stabilisatoren,
- Erhitzen der Blutprodukte in trockenem Zustand,
- Erhitzen der Blutprodukte in festem, nassem Zustand.

Derartige Verfahren sind bekannt und werden für sich nicht als patentfähig beansprucht. Nach fachmännischer Kenntnis ist darauf zu achten, die Inaktivierungsverfahren durch Erhitzen dahingehend auszuwählen, ob sie die potentielle Rest-Infektivität der Präparationen beseitigen und gleichzeitig ihre biologische Aktivität weitgehend erhalten.

Durch Erhitzen der Blutprodukte werden sowohl membranumhüllte als nicht nicht-membranumhüllte Viren angegriffen und inaktiviert.

Obwohl die Detergensbehandlung eine weitgehende Wirksamkeit ermöglicht, insbesondere auch Poxviren inaktiviert, können empfindliche Plasmaproteine weitgehend geschont werden. Auch der Zusatz einer toxischen Chemikalie (TNBP) ist nicht notwendig. Insbesondere wird eine mögliche chronische Toxizität von TNBP strikt vermieden.

Die Inaktivierungschemikalien müssen zwar entfernt werden, dennoch sind die Enfernungsschritte nicht so zeitraubend wie bei dem Stand der Technik. Beispielsweise muß keine Entfernung mittels C18-Harz vorgenommen werden (EP 366 946 B1, Erfinder: Bonomo).

Vorteilhafterweise wird bei der Hitzebehandlung so gearbeitet, daß einer Lösung an sich bekannte Stabilisatoren, wie α-Aminosäuren, Saccharose und Sorbit zugesetzt werden. Die Hitzebehandlung erfolgt bei 50 bis 65°C, üblicherweise über mehrere Stunden. Anschließend wird zur Verminderung der Viskosität die Lösung verdünnt und die Stabilizatoren durch Dialyse oder dergleichen entfernt.

Das Verfahren soll anhand mehrerer Beispiele erörtert werden.

### Vergleichsbeispiel 1: Gefrorenes Plasma, gewonnen beispielsweise aus Vollblutspenden nach Abtrennung der Zellen oder durch Plasmapherese, wird bei 20°C aufgetaut, der pH-Wert wird, falls erforderlich, auf 7,30 eingestellt und mit einer Stabilisatoren-Lösung (2 ml pro 100 ml Plasma) versetzt. Die Stabilisatoren-Lösung enthält pro 100 ml Wasser: 3,7 g CaCl₂, 2 H₂O, 15,0 g Arginin, 15,0 g Lysin, 18,0 g Na₃ citrat, 5,5 H₂O, pH 7,30. Anschließend werden 3 g Glycin und 600 g Saccharose pro 1 Plasma zugesetzt. Die Lösung wird gewogen und mit dem gleichen Gewicht Sorbit gemischt. Die Hitzebehandlung wird 10 Stunden bei 62 ± 0,5°C durchgeführt. Nach Verdünnen mit Dialysepuffer zur Verminderung der Viskosität werden die Stabilisatoren durch Dialyse, beispielsweise mittels Hohlfaserpatrone oder cross-flow Filtration an Membranen, entfernt. Der Dialysepuffer enthält pro 1 Wasser: 0,74 g CaCl₂, 2 H₂O, 3,0 g Arginin, 5,0 g Lysin, 3,6 g Na₃citrat, 5,5 H₂O, 3,0 g Glycin, 5,84 g NaCl. Die biologische Aktivität der folgenden Plasmaproteine beträgt mindestens 50 % des Wertes vor Hitzebehandlung: Fibrinogen, Faktor II, Faktor V, Faktor VII, Faktor VIII, Faktor IX, Faktor X, Faktor XI,Faktor XII, Faktor XIII, AT III, Plasminogen, Protein C, Protein S, α₂-Plasmin-Inhibitor. Die Anwesenheit aktivierter Gerinnungsfaktoren wird mit Hilfe von zwei empfindlichen Tests (nicht aktivierte partielle Thromboplastin-Zeit und Thrombin-Bildungszeit [C.W. Powse et al., Thrombosis Haemostasis (Stuttgart) 42, 1355 - 1367 (1979)] ausgeschlossen.

In einem getrennten Versuch wird zu 50 ml Lösung nach Zusatz aller Stabilisatoren bei Raumtemperatur 1 ml Hepatitis A-Virus-Suspension zugesetzt und eine Probe zur Messung des Virustiters gezogen. Anschließend wird über einen Zeitraum von 2 Stunden die Temperatur der Lösung gleichmäßig bis 62°C erhöht und dann 10 Stunden bei dieser Temperatur gehalten. Diese Vorgangsweise simuliert den Ablauf der Produktion. Nach der Dialyse wird eine weitere Probe zur Messung des Virustiters gezogen. Der Titer der Ausgangsprobe betrug 10^{4,9}, in der zweiten Probe war kein Virus nachweisbar.

### Vergleichbeispiels 2:

Analog zur Vorgangsweise in Vergleichsbeispiel 1 wird nach Zusatz aller Stabilisatoren bei Raumtemperatur 1 ml Sindbis-Virus-Suspension zugegeben und eine Probe zur Messung des Virustiters gezogen. Nach Hitzebehandlung und Dialyse, wie in Beispiel 1 beschrieben, wird eine weitere Probe zur Messung des Virustiters gezogen. Der Titer der Ausgangsprobe betrug 10^{5,6}, in der zweiten Probe war kein Virus nachweisbar.

### Vergleichsbeispiel 3:

Plasma nach Hitzebehandlung und Dialyse, wie in Vergleichsbeispiel 1 beschrieben, wird mit 1-O-n-Octyl-β-glucopyranosid (10g/l Plasma) versetzt und 20 min bei 25°C langsam gerührt. Anschließend wird mit Dialysepuffer (siehe Beispiel 1) im Volumenverhältnis 1:3 verdünnt und das Detergens durch Dialyse bzw. Diafiltration entfernt. Die biologische Aktivität der genannten Plasmaproteine beträgt mindestens 50 % des Wertes von unbehandeltem Plasma.

In einem getrennten Versuch wird zu 50 ml Plasma 1 ml Myxoma-Virus-Suspension zugesetzt und eine Probe zur Messung des Virustiters gezogen. Nach Zugabe von 0,5 g Detergens wird 15 min bei 25°C inkubiert und im Volumenverhältnis 1:10 mit Dialysepuffer verdünnt. Danach wird das Detergens durch Dialyse bzw. Diafiltration entfernt und eine Plasmaprobe zur Messung des Virustiters gezogen. Die Ausgangsprobe ergab einen Virustiter von 10^{6,2}, die zweite Probe ergab einen Titer von 10^{0,9},

### Beispiel 1:

Zu 1 l unbehandeltem Plasma werden Salze und Aminosäuren in der Menge wie für den Dialysepuffer des Vergleichsbeispiels 1, dann 15 g Triethylenglycol-mono-octylether/l Lösung zugesetzt und 10 min bei 25°C inkubiert. Nach Verdünnen mit Dialysepuffer im Volumenverhältnis 1:5 wird das Detergens durch Dialyse bzw. Diafiltration entfernt. Anschließend werden die Stabilisatoren für die Hitzebehandlung, wie in Beispiel 1 beschrieben, zugegeben und eine Hitzebehandlung in der angegebenen Weise durchgeführt. Danach werden die Stabilisatoren nach Verdünnen durch Dialyse bzw. Diafiltration entfernt. Die biologische Aktivität der genannten Plasmaproteine beträgt mindestens 50 % des Wertes vom Ausgangsplasma.

### Beispiel 2:

Zu 50 ml Plasma werden Salze und Aminosäuren in der Menge wie für den Dialysepuffer des Versgleichsbeispiels 1, dann 1 ml Pseudorabies-Virus-Suspension zugesetzt. Eine Probe wird zur Messung des Virustiters gezogen. Darauf wird 0,5 g 1-Thio-n-octyl-β-glucopyranosid zugegeben und 15 min bei Raumtemperatur inkubiert. Nach Verdünnung im Volumenverhältnis 1:10 wird das Detergens durch Dialyse bzw. Diafiltration entfernt. Eine Probe wird zur Messung des Virustiters gezogen. Der Titer der ersten Probe beträgt 10^{5,8}, in der zweiten Probe war der Virus nicht nachweisbar.

### Beispiel 3:

Analog zur Vorgangsweise in Beispiel 2 wird zu 50 ml Plasma mit Salzen und Aminosäuren 1 ml BVDV (bovine viral diarrhoea virus) zugesetzt. Eine Probe wird zur Messung des Virustiters gezogen. Danach wird 0,4 g N-(D-gluco-2,3,4-5,6-pantahydroxyhexyl)-N-methyloctanamid zugegeben und 10 min bei 25°C inkubiert. Nach Verdünnung im Volumenverhältnis 1:10 wird das Detergens durch Dialyse bzw. Diafiltration entfernt. Eine Probe wird zur Messung des Virustiters gezogen. Der Titer der ersten Probe beträgt 10^{5,1}, in der zweiten Probe war der Virus nicht nachweisbar.

Zweifach virusinaktiviertes Plasma ist per se ein wertvolles Therapeutikum mit sehr hoher Sicherheit gegenüber einer Übertragung von Viren aus Blut. Darüberhinaus können nach an sich bekannten Verfahren Plasmaderivate gleicher Sicherheit isoliert werden. Diese Vorgangsweise ist besonders vorteilhaft, weil die aufwendige Logistik der räumlichen Trennung von Zwischenstufen der Plasmafraktionierung vor und nach einer Virusinaktivierungsmaßnahme entfällt. Die folgenden Beispiel erläutern die Vorgangsweise.

### Beispiel 4:

Detergens-behandeltes oder zweifach virusinaktiviertes Plasma wird gefroren und zur Abtrennung eins Kryopräzipitates bei +4°C aufgetaut und bei dieser Temperatur zentrifugiert. Zur Gewinnung der Komponente 1 des Fibrinklebers wird das Kryopräzipitat mit eiskaltem Puffer gewaschen (Deutsches Arzneibuch 10, 3. Nachtrag 1994). Die Detergens- oder die sequentielle Detergens-/Hitzebehandlung kann auch mit dem gereinigten Präparat durchgeführt werden.

### Beispiel 5:

F VIII von Willebrand-Konzentrat wird beispielsweise durch Immunaffinitätschromatographie (EP-A-122 209) oder

Ionenaustauschchromatographie (DE 11 84 782) einer Lösung des Kryopräzipitates, hergestellt nach Beispiel 4, gereinigt. Die Detergens- oder sequentielle Hitze-/Detergensbehandlung kann auch mit dem gereinigten Präparat durchgeführt werden. Das Detergens kann auch im Verlauf der Chromatographie entfernt werden.

### Beispiel 6:

Die Faktoren des Prothrombinkomplexes, Faktor II, Faktor VII, Faktor IX und Faktor X, werden aus kryopräzipitatarmem Plasma durch Ionenaustauschchromatographie gewonnen [J. Heystek et al., Vox Sanguinis 25, 113 - 123 (1975)]. Die Detergens- oder die sequentielle Hitze-/Detergensbehandlung kann auch mir dem gereinigten Präparat durchgeführt werden. Das Detergens kann auch im Verlauf der Chromatographie entfernt werden.

### Beispiel 7:

Zur Verminderung des Thrombose-Risikos wird die Therapie von Hämophilie B vorzugsweise mit hoch gereinigtem F IX durchgeführt. Beispielsweise kann man eine solche Reinigung des Prothrombinkomplexes, gewonnen wie in Beispiel 9 beschrieben, mit Hilfe von Affinitätschromatographie durchführen [C. Michalski et al., Vox Sanguinis 55, 202 - 210 (1988), P.A. Feldmann et al., Blood Coagulation and Fibrinolysis 5, 939 - 948 (1994)]. Die Detergens- oder sequentielle Detergens-/Hitze-Behandlung kann auch mit dem gereinigten Präparat durchgeführt werden. Das Detergens kann auch im Verlauf der Chromatographie entfernt werden.

### Beispiels 8:

Aus dem Plasma nach Abtrennung des Kryopräzipitats und des Prothrombinkomplexes kann Antithrombin III durch Affinitätschromatographie gereinigt werden [R. Rosenberg und P.S. Damus, Journal of Biological Chemistry 248, 6490 - 6505 (1973)]. Die Detergens- oder sequentielle Hitze-/Detergens-Behandlung kann auch mit dem gereinigten Präparat durchgeführt werden. Das Detergens kann auch im Verlauf der Chromatographie entfernt werden.

### Beispiel 9:

Nach Abtrennung des Kryopräzipitats und gegebenenfalls weiterer Plasmaproteine kann aus Plasma gereinigtes Immunglobulin G durch eine der Varianten oder Modifikationen der Kälte-Alkohol-Fällung isoliert werden [E.J. Cohn et al., Journal of the American Chemical Society 68, 459 - 475 (1946), P. Kistler und H. Nitschmann, Vox Sanguinis 7, 414 - 424 (1962)]. Die Detergens- oder Hitze-/Detergens-Behandlung kann auch mit dem gereinigten Präparat durchgeführt werden. Das Detergens kann auch durch Fällung von Immunglobulin entfernt werden.

## Patentansprüche

1. Verfahren zur Herstellung eines virussicheren, therapeutischen, biologischen Präparates aus Humanplasma unter Anwendung eines Detergens, unter Erhaltung von mindestens 50% der biologischen Aktivität, an dessen Schluß die Detergentien entfernt werden, dadurch gekennzeichnet, daß unter Ausschluß von viruziden Chemikalien, wie Tri-n-butylphosphat, gearbeitet wird, daß als Detergentien nicht-ionische Tenside verwendet werden, die aus folgender Gruppe gewählt sind:
- Triethylenglycol-mono-octylether,
- N-(D-gluco-2,3,4,5,6-pentahydroxyhexyl)-N-methyloctanamid,
- 1-Thio-n-octyl-β-D-glucopyranosid,
wobei die Einwirkungsdauer des Detergens 5 bis 25 min, vorzugsweise 10 bis 20 min, und die Temperatur während der Detergensbehandlung 20° bis 30° C, vorzugsweise 25° C, beträgt.

2. Verfahren nach Anspruch 1, dadurch gekennzeichnet, daß nach Abschluß der Einwirkungsdauer die Konzentration des Detergens soweit verdünnt wird, daß die CMC (kritische Micellkonzentration) unterschritten und damit die Einwirkung abgebrochen wird.

3. Verfahren nach Anspruch 1 oder 2, dadurch gekennzeichnet, daß die Konzentration des Detergens zwischen 5g/l Plasma und 20 g/l Plasma beträgt.

4. Verfahren nach wenigstens einem der vorhergehenden Ansprüche, dadurch gekennzeichnet, daß das Detergens nach der Behandlung durch Dialyse oder Diafiltration entfernt wird.

5. Verfahren nach Anspruch 1 bis 4, dadurch gekennzeichnet, daß vor oder nach der Detergensbehandlung eine Hitzebehandlung, gegebenenfalls unter Anwendung üblicher Stabilisatoren, durchgeführt wird.

## Claims

1. Method of preparing a virally innocuous therapeutic biological preparation from human plasma using a detergent, retaining at least 50% of the biological activity, at the end of which the detergents are removed, characterised in that one operates with the exclusion of virucidal chemicals, such as tri-n-butylphosphate, that non-ionic surfactants which are selected from the following group:
- triethyleneglycol mono-octyl ether,
- N-(D-gluco-2,3,4,5,6-pentahydroxyhexyl)-N-methyl octanamide,
- 1-thio-n-octyl-β-D-glucopyranoside,
are used as detergents, the exposure time of the detergent being 5 to 25 min, preferably 10 to 20 min, and the temperature during detergent treatment being 20° to 30°C, preferably 25°C.

2. Method according to Claim 1, characterised in that, once the exposure time has ended, the concentration of the detergent is diluted to below the CMC (critical micelle concentration) and thus the exposure is terminated.

3. Method according to claim 1 or 2, characterised in that the concentration of the detergent is between 5 g/l plasma and 20 g/l plasma.

4. Method according to at least one of the foregoing claims, characterised in that the detergent is removed at the end of the treatment by dialysis or diafiltration.

5. Method according to Claims 1 to 4, characterised in that a heat treatment is carried out before or after the detergent treatment, optionally using conventional stabilisers.

## Revendications

1. Procédé de fabrication d'une préparation biologique thérapeutique exempte de virus à partir de plasma humain en utilisant un détergent, en conservant au moins 50% de l'activité biologique, à la fin duquel les détergents sont éliminés, caractérisé en ce que
le procédé est mis en oeuvre en excluant tous produits viricides, comme du phosphate de tri-n-butyle
des dérivés tensio-actifs non ioniques choisis dans le groupe suivant sont employés comme détergents:
- le mono-octyléther de triéthylèneglycol,
- le N-(D-gluco-2, 3, 4, 5, 6-pentahydroxyhexyl) -N-méthyloctanamide,
- le 1-thio-n-octyl-β-D-glucopyranoside,
la durée d'intervention du détergent étant de 5 à 25 minutes, de préférence de 10 à 20 minutes, et la température étant de 20° à 30° C, de préférence de 25°C pendant le traitement au détergent.

2. Procédé selon la revendication 1, caractérisé en ce que la concentration en détergent est diluée, après la fin de la durée d'intervention, jusqu'à ce que sa valeur passe au-dessous de la concentration critique de micelle (ou CMC) et que l'intervention soit interrompue de cette manière.

3. Procédé selon la revendication 1 ou 2, caractérisé en ce que la concentration en détergent est comprise entre 5 g/l de plasma et 20 g/l de plasma.

4. Procédé selon l'une au moins des revendications précédentes, caractérisé en ce que le détergent est éliminé par dialyse ou diafiltration, après le traitement.

5. Procédé selon l'une quelconque des revendications 1 à 4, caractérisé en ce qu'un traitement thermique est exécuté, éventuellement en utilisant des stabilisants habituels, avant ou après le traitement au détergent.
